# EUROPEAN PATENT SPECIFICATION

(11) **EP 3 560 464 B1**
(45) Date of publication and mention of the grant of the patent: **25.08.2021**
(21) Application number: 19167739.2
(22) Date of filing: 08.04.2019
(51) Int. Cl.: A61F 13/49, A61F 13/511

(54) **PANTS-SHAPED ABSORBENT ARTICLE AND METHOD FOR MANUFACTURING PANTS-SHAPED ABSORBENT ARTICLE**
HOSENFÖRMIGER ABSORBIERENDER ARTIKEL UND VERFAHREN ZUR HERSTELLUNG EINES HOSENFÖRMIGEN ABSORBIERENDEN ARTIKELS
ARTICLE ABSORBANT EN FORME DE CULOTTE ET PROCÉDÉ DE FABRICATION D'UN ARTICLE ABSORBANT EN FORME DE CULOTTE

(30) Priority: 27.04.2018 JP 2018086106
(43) Date of publication of application: 30.10.2019
(73) Proprietor: Unicharm Corporation, Shikokuchuo-shi, Ehime 799-0111 (JP)
(72) Inventor: MUKAI, Hirotomo, Kanonji-shi, Kagawa 769-1602 (JP)
(74) Representative: Dolleymores

(56) References cited:
- EP-A1- 3 305 261
- WO-A1-2014/002440
- WO-A1-2015/068486
- WO-A1-2018/043080
- JP-A- 2009 297 299

## Description

### [Technical Field]

The present invention relates to a pants-shaped absorbent article and a method for manufacturing a pants-shaped absorbent article.

### [Background Art]

A conventionally known pants-shaped absorbent article includes an absorbent main body that has an absorbent core, and an exterior member that is joined to the absorbent main body and is provided with a waist opening and a pair of leg openings. Patent Document 1 discloses a pants-shaped absorbent article that includes an exterior member in which an inner sheet is arranged on the skin side of an upper front portion, an outer-layer sheet forms the outer surface, and an intermediate sheet is fixed between the inner sheet and the outer-layer sheet.

### [Citation List]

### [Patent Literature]

[Patent Document 1] Japanese Patent Application Publication No. 2013-172861
JP 2009297299, WO 2015/068486 A1, WO 2018/043080 A1 and WO 2014/002440 A1 all disclose pants-shaped absorbent articles.

### [Summary of Invention]

### [Technical Problem]

However, in the pants-shaped absorbent article disclosed in Patent Document 1, the outer-layer sheet and the intermediate sheet are not joined to each other. For this reason, when the absorbent article is pulled up by a wearer for example, the un-joined portion is likely to stretch in the vertical direction (up-down direction), and if force is applied to portions by fingers or the like in this stretched state, there is a risk of the sheet being ripped. Also, in the case where three sheets are overlaid on each other, and an elastic member is arranged between two of such sheets, if all of the sheets are joined using an adhesive, there is a risk of the sheets becoming hard, whereas if all of the sheets are joined by welding, there is a risk of the elastic member becoming ruptured.

The present invention was achieved in light of conventional problems such as that described above, and an aspect of the present invention is to provide a pants-shaped absorbent article that is soft and is reinforced without rupture of an elastic member.

### [Solution to Problem]

The present invention provides the pants-shaped absorbent article of independent claim 1 and the method for manufacturing a pants-shaped absorbent article of independent claim 12. The dependent claims specify preferred but optional features.

A main aspect of the present invention for achieving the above-described aspect is a pants-shaped absorbent article
having an up-down direction, a left-right direction, and a front-back direction that intersect each other, and
having a waist opening and a pair of leg openings,
the pants-shaped absorbent article including:
an absorbent main body having an absorbent core; and
an exterior member joined to the absorbent main body,
the exterior member having an overlaid portion in which a first sheet member, a second sheet member, and a third sheet member are overlaid in order from a skin side to a non-skin side in a thickness direction,
the first sheet member and the second sheet member being joined by being intermittently welded in a plurality of welded portions,
an elastic member being arranged between the second sheet member and the third sheet member,
the second sheet member and the third sheet member being joined using an adhesive; wherein
a length of the second sheet member in the up-down direction is shorter than a length of the first sheet member in the up-down direction, and
the plurality of welded portions are formed so as to straddle at least one end portion in the up-down direction of the second sheet member in a portion in the left-right direction.

Features of the present invention other than the above will become clear by reading the description of the present specification with reference to the accompanying drawings.

### [Advantageous Effects of Invention]

According to the present invention, it is possible to achieve softness and reinforcement without rupture of an elastic member.

### [Brief Description of the Drawings]

FIG. 1 is a schematic perspective view of a diaper 1 as seen from a stomach side.
FIG. 2 is a plan view of the diaper 1 in an unfolded and stretched state.
FIG. 3 is a schematic cross-sectional view taken along A-A in FIG. 2.
FIG. 4 is a diagram showing an example of a pattern of fused portions 31a provided in the surface of a front inner-layer sheet 31.
FIG. 5 is a diagram showing an example of a pattern of fused portions 32a provided in the surface of a front reinforcing sheet 32.
FIG. 6 is a diagram showing an example of a pattern of welded portions 60a formed in a composite sheet 60.
FIG. 7 is a diagram for illustrating configurations of composite sheets 60 and 70.

### [Description of Embodiments]

At least the following matters will become clear with the description of this specification and the attached drawings.

A pants-shaped absorbent article
having an up-down direction, a left-right direction, and a front-back direction that intersect each other, and
having a waist opening and a pair of leg openings,
the pants-shaped absorbent article including:
an absorbent main body having an absorbent core; and
an exterior member joined to the absorbent main body,
the exterior member having an overlaid portion in which a first sheet member, a second sheet member, and a third sheet member are overlaid in order from a skin side to a non-skin side in a thickness direction,
the first sheet member and the second sheet member being joined by being intermittently welded in a plurality of welded portions,
an elastic member being arranged between the second sheet member and the third sheet member,
the second sheet member and the third sheet member being joined using an adhesive.

According to this pants-shaped absorbent article, three sheet members are overlaid on each other, thus making it possible to reinforce a portion that is easily torn when a wearer or caregiver pulls up the absorbent article. Also, the first sheet member and the second sheet member are intermittently welded to each other, thus making it possible to maintain a soft texture. Also, the second sheet member and the third sheet member are joined by an adhesive, thus making it possible to prevent rupturing of an elastic member.

In such a pants-shaped absorbent article,
a length of the second sheet member in the up-down direction is shorter than a length of the first sheet member in the up-down direction, and
the plurality of welded portions are formed so as to straddle at least one end portion in the up-down direction of the second sheet member in a portion in the left-right direction.

According to this pants-shaped absorbent article, the second sheet member can be prevented from peeling, and it is possible to reliably maintain strength up to the ends in the up-down direction.

In such a pants-shaped absorbent article, it is desirable that
the plurality of welded portions are arranged in a staggered manner in the up-down direction and the left-right direction.

According to this pants-shaped absorbent article, it is possible to eliminate a large un-joined portion, thus making it less likely for a finger to penetrate (less likely for rupturing to occur). Also, portions do not become excessively hard, and overall softness is realized.

In such a pants-shaped absorbent article, it is desirable
that the first sheet member and the second sheet member each have a plurality of fused portions at a surface, and
that a ratio of an area of the welded portions provided per unit area of the overlaid portion
is smaller than at least either one of
a ratio of an area of the fused portions provided per unit area of the first sheet member and
a ratio of an area of the fused portions provided per unit area of the second sheet member.

According to this pants-shaped absorbent article, it is possible to further maintain the soft texture of the sheets, and to improve the fit to the wearer.

In such a pants-shaped absorbent article, it is desirable
that the first sheet member and the second sheet member each have a plurality of fused portions at a surface, and
that an area of each of the welded portions
   is smaller than at least either one of
   an area of each of the fused portions in the first sheet member and
   an area of each of the fused portions in the second sheet member.

According to this pants-shaped absorbent article, it is possible to further maintain the soft texture of the sheets, and to improve the fit to the wearer.

In such a pants-shaped absorbent article, it is desirable that
a ratio of an area of the welded portions provided per unit area of a region in which the exterior member and the absorbent main body are overlapped
   is smaller than
a ratio of an area of the welded portions provided per unit area of another region.

According to this pants-shaped absorbent article, the portion overlapped with the absorbent main body is not likely to become torn during use, thus making it possible to reduce the area ratio of the welded portions . Accordingly, it is possible to further improve the softness.

In such a pants-shaped absorbent article, it is desirable
that a plurality of the elastic members are arranged with gaps in the up-down direction,
that the adhesive is applied to each of the elastic members, and
that a portion in which the second sheet member and the third sheet member are not joined by the adhesive is provided between adjacent elastic members.

According to this pants-shaped absorbent article, the adhesive can be used efficiently. Also, softness can be further improved.

In such a pants-shaped absorbent article, it is desirable
that the exterior member includes
   a first stretchy region that extends along an edge of the waist opening and
   a pair of second stretchy regions that respectively extend along edges of the pair of leg openings, and
that with respect to the up-down direction, at least a portion of the overlaid portion is provided between the first stretchy region and the pair of second stretchy regions.

According to this pants-shaped absorbent article, it is possible to reinforce a portion that is likely to be subjected to force during use.

In such a pants-shaped absorbent article, it is desirable
that a plurality of waist elastic members that are arranged with gaps in the up-down direction is provided in the first stretchy region, and
that a gap between adjacent elastic members in the overlaid portion is larger than a gap between adjacent waist elastic members in the first stretchy region.

According to this pants-shaped absorbent article, the arrangement interval between the elastic members (waist elastic members) on the waist opening side is reduced, thus making it difficult to insert a finger when pulling up the absorbent article, and also making it possible to suppress twisting of the absorbent article in the up-down direction. As for the overlaid portion, it is possible to reduce a feeling of being constricted.

In such a pants-shaped absorbent article, it is desirable
that the exterior member includes
   a front waist portion that is to be arranged on a stomach side of a wearer, and
   a back waist portion that is to be arranged on a back side of the wearer, and
that the overlaid portion is provided in at least either one of the front waist portion and the back waist portion.

According to this pants-shaped absorbent article, the waist portion can be reinforced.

In such a pants-shaped absorbent article, it is desirable
that the overlaid portion is provided in both the front waist portion and the back waist portion, and
that an up-down-direction length of the overlaid portion of the back waist portion is larger than an up-down-direction length of the overlaid portion of the front waist portion.

According to this pants-shaped absorbent article, the back side has a large region that is easily grabbed with fingers when the absorbent article is pulled up (pull-up is difficult due to the protruding buttocks portion), and therefore this region can be effectively reinforced.

In such a pants-shaped absorbent article, it is desirable
that the third sheet member is folded back downward on the skin side in the thickness direction at an edge of the waist opening, and
that an upper end of the second sheet member in the up-down direction is located higher than a lower end of a folded portion of the third sheet member.

According to this pants-shaped absorbent article, reinforcement can be more reliably achieved.

A method for manufacturing a pants-shaped absorbent article having a waist opening and a pair of leg openings,
the pants-shaped absorbent article including:
an absorbent main body having an absorbent core; and
an exterior member joined to the absorbent main body,
the method including:
   intermittently welding a first sheet member and a second sheet member with a plurality of welded portions;
   arranging an elastic member between a surface of the second sheet member and a third sheet member and joining the second sheet member and the third sheet member with an adhesive,
      the surface of the second sheet member being located on an opposite side of the first sheet member; and
   forming an overlaid portion in which the first sheet member, the second sheet member, and the third sheet member are overlaid in order from a skin side to a non-skin side in a thickness direction of the exterior member.

### Embodiment

### Basic configuration of diaper 1

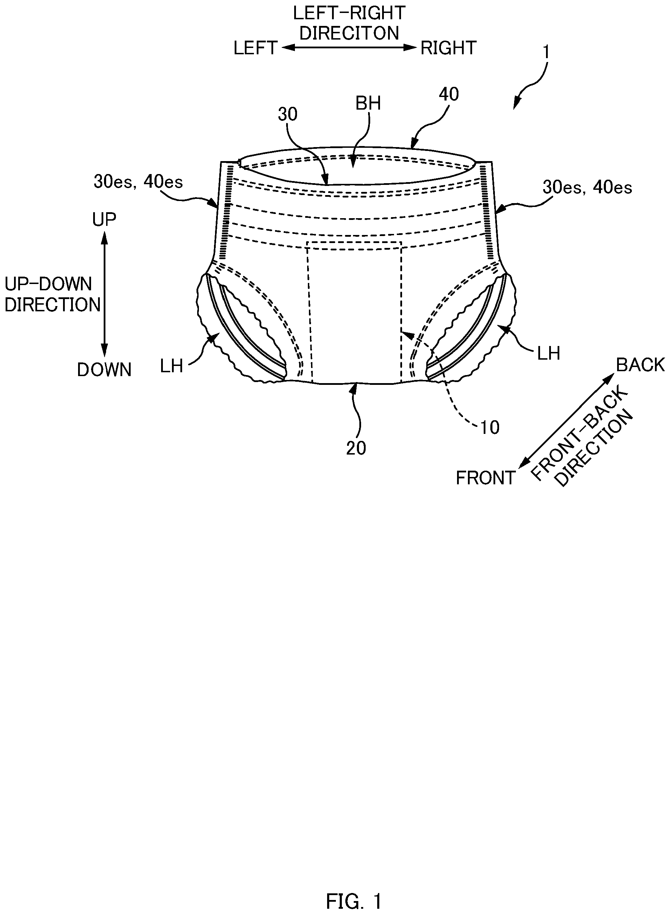

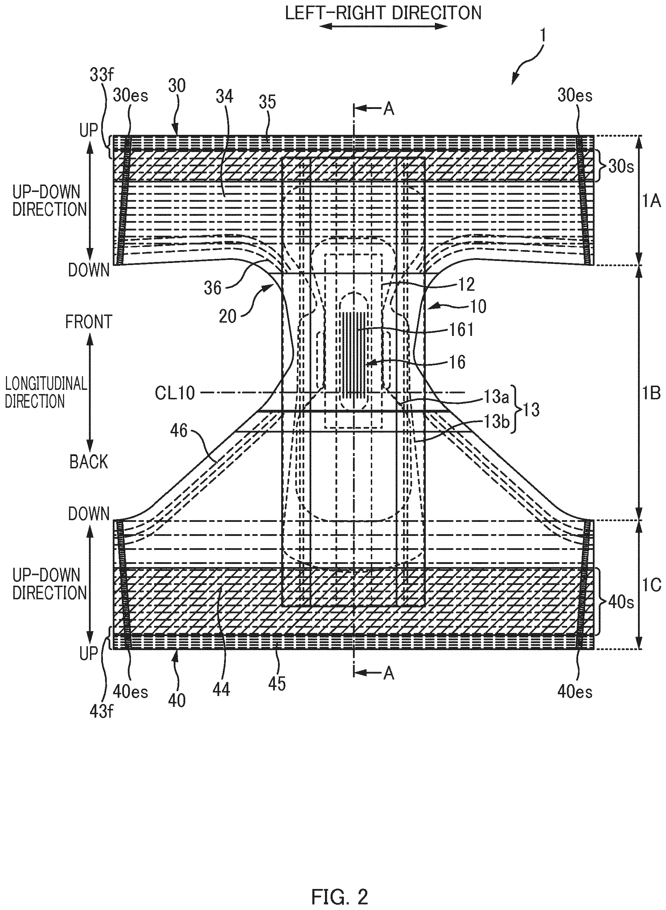

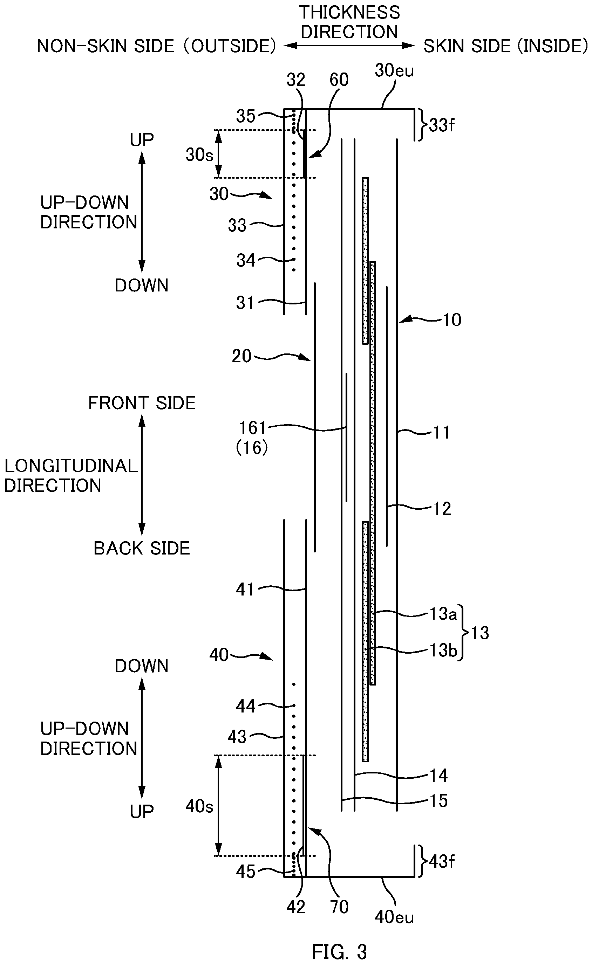

The following describes the basic configuration of a pull-on disposable diaper 1 (hereinafter, also called the "diaper 1") as one example of a pants-shaped absorbent article. FIG. 1 is a schematic perspective view of the diaper 1 as seen from a front side. FIG. 2 is a plan view of the diaper 1 in an unfolded and stretched state. FIG. 3 is a schematic cross-sectional view taken along A-A in FIG. 2. Note that the "stretched state" in FIG. 2 refers to a state in which the product (diaper 1) is stretched so as to eliminate wrinkles, or more specifically is stretched such that the lengths of the constituent members of the diaper 1 (e.g., a later-described front panel portion 30) match or are close to the dimensions of the members on their own.

In the pants-shaped state in FIG. 1, the diaper 1 has an up-down direction, a left-right direction (also called a "width direction"), and a front-back direction as three directions that are orthogonal to each other. Hereinafter, in the up-down direction, the side corresponding to the wearer's trunk side is the "upper side", and the side corresponding to the wearer's crotch side is the "lower side" . In the front-back direction, the side corresponding to the wearer's stomach side is the "front side", and the side corresponding to the wearer's back side is the "back side". Also, in the left-right direction, the right side and the left side when viewing the diaper 1 from the front are respectively the "right side" and the "left side".

Also, in the unfolded state in FIGS. 2 and 3, the diaper 1 has a longitudinal direction, a left-right direction, and a thickness direction as three directions that are orthogonal to each other. Note that the left-right direction (width direction) in the unfolded state is the same direction as the left-right direction in the pants-shaped state (FIG. 1). Also, the longitudinal direction in the unfolded state is a direction that conforms to the up-down direction in the pants-shaped state. Moreover, as shown in FIG. 3, in the "thickness direction" that is orthogonal to the left-right direction and the up-down direction (longitudinal direction), the side that comes into contact with the target's (wearer's) skin is the "skin side", and the opposite side is the "non-skin side".

The diaper 1 of the present embodiment has an absorbent main body 10, a crotch exterior portion 20, a front panel portion 30, and a back panel portion 40. Note that the crotch exterior portion 20, the front panel portion 30, and the back panel portion 40 correspond to exterior members and are joined to the absorbent main body 10. As shown in FIG. 2, a front waist portion 1A, a crotch portion 1B, and a back waist portion 1C are constituted by these exterior members. Note that although the exterior members are constituted by three members (the crotch exterior portion 20, the front panel portion 30, and the back panel portion 40) in the diaper 1 of the present embodiment, there is no limitation to this. For example, the crotch exterior portion 20, the front panel portion 30, and the back panel portion 40 may be a single member. Also, the crotch exterior portion 20 may be omitted.

The absorbent main body 10 has a function of absorbing excrement such as urine, is approximately rectangular in a plan view as shown in FIG. 2, and is arranged at the center in the left-right direction with the longitudinal direction conforming to the up-down direction of the diaper 1. As shown in FIG. 3, the absorbent main body 10 has a liquid-permeable top sheet 11, a liquid-permeable second sheet 12, an absorbent core 13, a liquid-impermeable back sheet 14, and a crotch-elastic-member covering sheet 15 in this order beginning from the skin side in the thickness direction (the side that comes into contact with the wearer).

Hydrophilic spunbond nonwoven fabric or the like is one example of the top sheet 11, hydrophilic air-through nonwoven fabric or the like is one example of the second sheet 12, and a polyethylene film or the like is one example of the back sheet 14. The crotch-elastic-member covering sheet 15 may be a liquid-permeable sheet or a liquid-impermeable sheet, and one example is hydrophobic spunbond nonwoven fabric. Note that the second sheet 12 serves roles such as improving absorption through a density gradient with the top sheet 11 and preventing the backflow of excrement, but an aspect is possible in which the diaper 1 does not have the second sheet 12.

The absorbent core 13 is a member that absorbs and holds excrement such as urine, and is formed by liquid absorbent fibers such as pulp mixed with the superabsorbent polymer (SAP) for example. Note that the absorbent core 13 may be covered by a liquid-permeable sheet made of tissue or the like. Also, the absorbent core 13 has a two-layer structure and includes: an upper layer core 13a; and a lower layer core 13b that is arranged on the non-skin side in the thickness direction relative to the upper layer core 13a.

Also, in a central portion with respect to the width direction and the longitudinal direction, the absorbent main body 10 has a crotch elastic portion 16 that stretches and contracts in the longitudinal direction. The crotch elastic portion 16 is formed by providing a plurality of crotch elastic members 161 (e.g., elastic strings) with gaps therebetween in the width direction. The crotch elastic members 161 are attached between the back sheet 14 and the crotch-elastic-member covering sheet 15 in a state of being stretched in the longitudinal direction.

Note that although the absorbent core 13 of the present embodiment has a two-layer structure, there is no limitation to this, and the absorbent core 13 may have a one-layer structure. Also, an aspect is possible in which the crotch elastic portion 16 and the crotch-elastic-member covering sheet 15 are not provided in the diaper 1.

The crotch exterior portion 20 is a portion that is located at the wearer's crotch (a portion that constitutes a portion of the crotch portion 1B) when the diaper 1 is worn. In the present embodiment, the crotch exterior portion 20 is formed by spunbond nonwoven fabric or the like.

The front panel portion 30 is a portion that is located around the wearer's waist on the front side (a portion that mainly constitutes the front waist portion 1A) when the diaper 1 is worn. The front panel portion 30 has a front inner-layer sheet 31 and a front outer-layer sheet 33 that are overlaid on each other in the thickness direction. As shown in FIG. 3, the front inner-layer sheet 31 is arranged on the skin side in the thickness direction, and the front outer-layer sheet 33 is arranged on the non-skin side in the thickness direction. The upper end portion of the front outer-layer sheet 33 protrudes upward beyond the upper end portion of the front inner-layer sheet 31, and this protruding portion is folded back downward to the skin side in the thickness direction at a position corresponding to an upper end 30eu of the front panel portion 30, thus forming a folded portion 33f. The folded portion 33f of the diaper 1 of the present embodiment has a length of approximately 30 mm in the up-down direction. The upper end 30eu of the front panel portion 30 is covered by the folded portion 33f, thus suppressing the case where the upper end edge of the front panel portion 30 (i.e., the waist opening BH) digs into the wearer's skin when the diaper 1 is worn, and also making it possible to suppress discomfort at the front waist portion 1A.

Also, in the diaper 1 of the present embodiment, a front reinforcing sheet 32 is provided between the front inner-layer sheet 31 and the front outer-layer sheet 33 of the front panel portion 30. Hereinafter, the portion in which three sheets (the front inner-layer sheet 31, the front reinforcing sheet 32, and the front outer-layer sheet 33) are overlaid in the front panel portion 30 will also be called an overlaid portion 30s. Also, a sheet obtained by joining the front inner-layer sheet 31 and the front reinforcing sheet 32 will also be called a composite sheet 60. Details of the overlaid portion 30s and the composite sheet 60 will be described later.

Also, elastic members such as elastic strings are provided between the composite sheet 60 (the front inner-layer sheet 31 and the front reinforcing sheet 32) and the front outer-layer sheet 33 in the thickness direction. In the present embodiment, as shown in FIGS. 2 and 3, a plurality of front waist elastic members 34 are provided in a region between the right end portion and the left end portion of the front panel portion 30 with respect to the left-right direction. The front waist elastic members 34 are joined to and sandwiched between the composite sheet 60 and the front outer-layer sheet 33 in a state of being arranged with gaps therebetween in the up-down direction and stretched in the lateral direction with a predetermined stretch factor. Providing the front waist elastic members 34 gives widthwise stretchability to the front panel portion 30 of the diaper 1. Note that the "stretch factor" of the elastic member indicates the extent of stretch relative to the value of 1 for the natural length of the elastic member (elastic string). For example, in the case where the stretch factor is 1.2, the elastic member stretches by a factor of 0.2 from the natural length.

Also, a plurality of waist elastic members 35 are provided in a region extending along the edge of the waist opening BH of the front panel portion 30 (this region corresponds to a first stretchy region), and are similarly arranged with gaps therebetween in the up-down direction. As shown in FIGS. 2 and 3, the gap in the up-down direction (pitch) between adjacent front waist elastic members 34 is larger than the gap in the up-down direction between adjacent waist elastic members 35. In the present embodiment, the gap between waist elastic members 35 that are adjacent in the up-down direction is 4 mm (pitch of 4 mm), whereas the gap between front waist elastic members 34 that are adjacent in the up-down direction is 10 mm (pitch of 10 mm).

In this way, the gaps between the waist elastic members 35 that are closer to the waist opening BH are reduced, thus making it difficult to insert fingers when pulling up the diaper 1, and also making it possible to suppress twisting of the diaper 1 in the up-down direction. Also, increasing the pitch of the front waist elastic members 34 makes it possible to reduce the feeling of being constricted.

The back panel portion 40 is a portion that is located around the wearer's waist on the back side (a portion that mainly constitutes the back waist portion 1C) when the diaper 1 is worn. The structure of the back panel portion 40 is substantially similar to that of the front panel portion 30. Specifically, the back panel portion 40 has a back inner-layer sheet 41 and a back outer-layer sheet 43 that are overlaid on each other in the thickness direction. The upper end portion of the back outer-layer sheet 43 protrudes upward beyond the upper end portion of the back inner-layer sheet 41, and this protruding portion is folded back downward On the skin side in the thickness direction at a position corresponding to an upper end 40eu of the back panel portion 40, thus forming a folded portion 43f. The length of the folded portion 43f in the up-down direction is the same as the length of the folded portion 33f in the up-down direction (approximately 30 mm) . Accordingly, this suppresses a case where the upper end edge of the back panel portion 40 (i.e., the waist opening BH) digs into the wearer's skin when the diaper 1 is worn, and also makes it possible to suppress discomfort at the back waist portion 1C.

Also, similarly to the front panel portion 30, the back panel portion 40 of the present embodiment is provided with a back reinforcing sheet 42 between the back inner-layer sheet 41 and the back outer-layer sheet 43. Hereinafter, the portion in which three sheets (the back inner-layer sheet 41, the back reinforcing sheet 42, and the back outer-layer sheet 43) are overlaid in the back panel portion 40 will also be called an overlaid portion 40s. Also, a sheet obtained by joining the back inner-layer sheet 41 and the back reinforcing sheet 42 will also be called a composite sheet 70. The overlaid portion 40s has a configuration similar to that of the overlaid portion 30s, but has a different formation region width (length in the up-down direction).

Also, as shown in FIG. 2, a plurality of back waist elastic members 44 are provided in a region between the right end portion and the left end portion of the back panel portion 40 with respect to the left-right direction. The back waist elastic members 44 are joined to and sandwiched between the composite sheet 70 (the back inner-layer sheet 41 and the back reinforcing sheet 42) and the back outer-layer sheet 43 in a state of being arranged with gaps therebetween in the up-down direction and stretched in the left-right direction with a predetermined stretch factor. Providing the back waist elastic members 44 gives widthwise stretchability to the back panel portion 40 of the diaper 1. Also, a plurality of waist elastic members 45 are provided in a region extending along the edge of the waist opening BH of the back panel portion 40 (this region corresponds to a first stretchy region), and are similarly arranged with gaps therebetween in the up-down direction.

Note that the gaps between back waist elastic members 44 that are adjacent in the up-down direction and the gaps between adjacent waist elastic members 45 are respectively the same as the gaps between adjacent waist elastic members 34 and the gaps between adjacent waist elastic members 35.

Also, as shown in FIG. 2, leg elastic members 36 and 46 such as elastic strings are also respectively provided in the regions extending along the edges of the pair of leg openings HL in the front panel portion 30 and back panel portion 40 (these regions corresponding to second stretchy regions) . The leg elastic members 36 and 46 are attached in a stretched state between the front inner-layer sheet 31 and the front outer-layer sheet 33 and between the back inner-layer sheet 41 and the back outer-layer sheet 43 respectively. Accordingly, the front panel portion 30 and the back panel portion 40 fit around the wearer's legs.

In the process for manufacturing the diaper 1, the various members are arranged in the unfolded state in FIG. 2 and joined to each other. For example, an adhesive (e.g., hot-melt adhesive HMA) is applied in a pattern along the leg elastic members 36 and 46 on the front inner-layer sheet 31 and the back inner-layer sheet 41. Also, an adhesive is applied to the entire surface on the front outer-layer sheet 33 side and the back outer-layer sheet 43 side in a region from the lower portions of the front waist portion 1A and the back waist portion 1C to the end portions of the crotch portion 1B in the left-right direction. These sheets are then overlaid on each other so as to be fixed together.

Note that in the front panel portion 30, the composite sheet 60 and the front outer-layer sheet 33 are joined by an adhesive that has been directly applied to the front waist elastic members 34 and the waist elastic members 35. For this reason, a portion in which the composite sheet 60 and the front outer-layer sheet 33 are not joined by an adhesive is provided between adjacent front waist elastic members 34. A similar configuration is employed on the back side (back panel portion 40) as well.

In the present embodiment, the hot-melt adhesive HMA is applied using a V-slot nozzle method, a spiral method, and a slot coater method.

The V-slot nozzle method is used to fix the front waist elastic members 34, the waist elastic members 35, the back waist elastic members 44, and the waist elastic members 45. With this method, the adhesive is applied over the entire length of these elastic members in the length direction thereof.

The spiral method is used to fix the folded portions 33f and 43f, the leg elastic members 36 and 46, and the like, and the adhesive is applied in a pattern extending to the inward sides of side joining portions (front side joining portion 30es and back side joining portion 40es) so as to not be overlapped with the side joining portions.

The slot coater method is for applying an adhesive in a fixed width over the entire surface or in a linear manner.

Note that the composite sheets 60 and 70 are joined by intermittently welding two sheets thereof (described later).

After the various members have been arranged (joined) in the unfolded state in FIG. 2, the absorbent main body 10 is folded one time at a folding position corresponding to a predetermined position CL10 in the longitudinal direction (up-down direction) of the absorbent main body 10. The front panel portion 30 and the back panel portion 40, which face each other in this folded state, are joined by welding or the like to the front side joining portions 30es and the back side joining portions 40es. Accordingly, the front panel portion 30 and the back panel portion 40 are connected to form a ring-like shape, thus forming the waist opening BH and the pair of leg openings LH as shown in FIG. 1, thereby obtaining the diaper 1 in the pants-shaped state.

### Overlaid portion

As previously described, in the diaper 1 of the present embodiment, the front panel portion 30 and the back panel portion 40 respectively include the overlaid portions 30s and 40s in which three sheets are overlaid on each other. Note that the configurations of the front panel portion 30 and the back panel portion 40 are substantially similar to each other. The following mainly describes the configuration of the front panel portion 30.

If the front panel portion 30 were constituted by two sheets, namely the front inner-layer sheet 31 and the front outer-layer sheet 33, there would be a risk of a finger penetrating between the two sheets when the wearer of the diaper 1 or the like pulls up the diaper 1. In view of this, in the present embodiment, the front reinforcing sheet 32 is arranged between the front inner-layer sheet 31 and the front outer-layer sheet 33, thus providing the overlaid portion 30s in which three sheets are overlaid on each other. It should be noted that if all three sheets are joined by an adhesive, there is a risk of hardening, and if all three sheets are joined by welding, there is a risk of damage to elastic members (e.g., the front waist elastic members 34).

For this reason, in the present embodiment, the front inner-layer sheet 31 and the front reinforcing sheet 32 are joined by welding, and the front reinforcing sheet 32 and the front outer-layer sheet 33 are joined using an adhesive. More specifically, first, the front inner-layer sheet 31 and the front reinforcing sheet 32 are intermittently welded, thus forming the composite sheet 60. Next, the front waist elastic members 34 having an adhesive applied thereon are arranged between the composite sheet 60 and the front outer-layer sheet 33 with gaps therebetween in the up-down direction, and the composite sheet 60 and the front outer-layer sheet 33 are joined via this adhesive.

First, the sheet members that constitute the overlaid portion 30s of the front panel portion 30 (i.e., the front inner-layer sheet 31, the front reinforcing sheet 32, and the front outer-layer sheet 33) will be described. In the diaper 1 of the present embodiment, spunbond nonwoven fabric is used for the front inner-layer sheet 31 and the front outer-layer sheet 33, and SMS (spunbond/melt-blown/spunbond) nonwoven fabric is used for the front reinforcing sheet 32. A plurality of emboss portions for maintaining strength are then provided in the surfaces of these nonwoven fabric sheets. In the following description, the emboss portions provided in the surfaces of the nonwoven fabric sheets will be called fused portions.

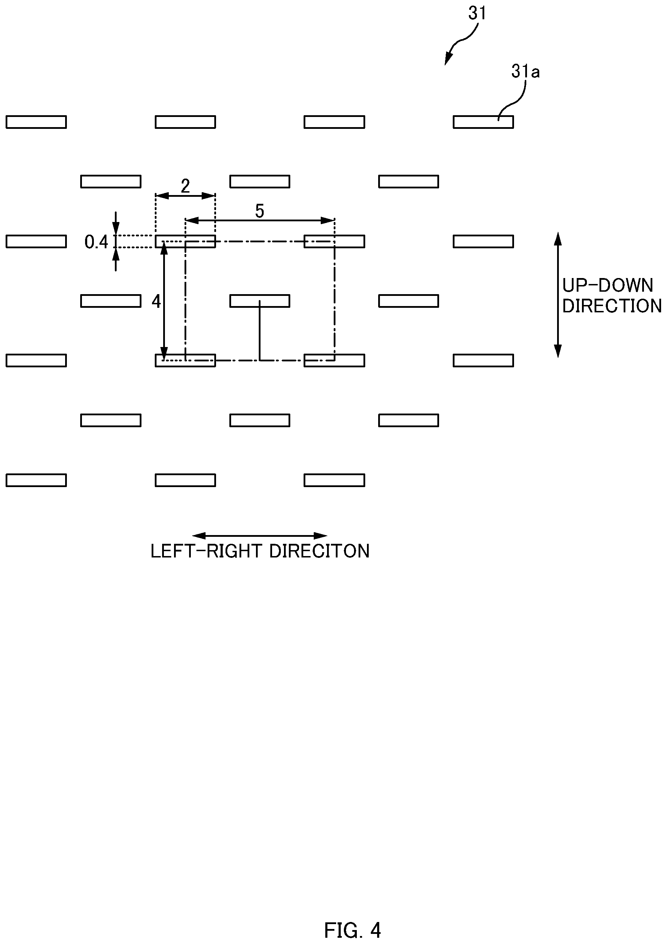

FIG. 4 is a diagram showing an example of a pattern of fused portions 31a provided in the surface of the front inner-layer sheet 31. The front inner-layer sheet 31 is made of spunbond nonwoven fabric, and laterally elongated emboss portions (fused portions 31a) are formed regularly over the entire region of the surface of the front inner-layer sheet 31 as shown in FIG. 4. The pitch of the fused portions 31a is desirably approximately 2 to 6 mm in the up-down direction and the left-right direction, and within this range, it is more likely to maintain both the strength and the softness of the nonwoven fabric. The fused portions 31a of the present embodiment are shaped as rectangles that are 2.0 mm in the left-right direction and 0.4 mm in the up-down direction, and the fused portions 31a each have an area of 0.8 mm² (=2.0 mm × 0.4 mm). Also, the fused portions 31a are formed in a staggered pattern with a pitch of 5 mm in the left-right direction and a pitch of 4.0 mm in the up-down direction. Accordingly, it is possible to maintain both the strength and the softness of the nonwoven fabric.

Also, as shown in FIG. 4, the fused portions 31a are provided at a rate of 2 per area of 4 mm × 5 mm. Accordingly, the ratio of the area of the fused portions 31a provided per unit area of the front inner-layer sheet 31 (hereinafter, called the area ratio) is (0.8 mm²) × (4 mm × 5 mm) × 100 = 8.0%.

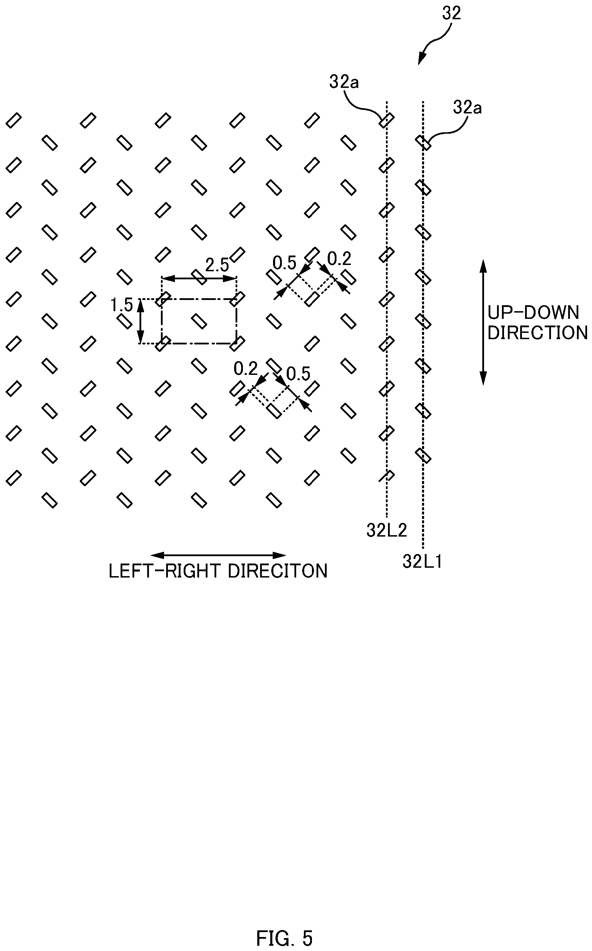

FIG. 5 is a diagram showing an example of a pattern of fused portions 32a provided in the surface of the front reinforcing sheet 32. The front reinforcing sheet 32 is made of SMS nonwoven fabric, and emboss portions (fused portions 32a) are provided regularly over the entire region of the surface of the front reinforcing sheet 32 as shown in FIG. 5.

The fused portions 32a of the front reinforcing sheet 32 are rectangles having long sides of 0 .5 mm and short sides of 0.2 mm, forming in a pattern. The fused portions 32a are arranged side-by-side in the up-down direction to form dot lines 32L1 and dot lines 32L2 in the front reinforcing sheet 32. In the dot lines 32L1, the fused portions 32a are arranged side-by-side with gaps therebetween in the up-down direction in a pattern in which the long sides are rotated 45 degrees clockwise from the state of conforming to the left-right direction. In the dot lines 32L2, the fused portions 32a are arranged side-by-side with gaps therebetween in the up-down direction in a pattern in which the long sides are rotated 45 degrees counter-clockwise from the state of conforming to the left-right direction. Note that with respect to the up-down direction, the fused portions 32a in the dot lines 32L2 are located between (at an intermediate position between) fused portions 32a that are adjacent in the up-down direction in the dot lines 32L1. These dot lines 32L1 and dot lines 32L2 are alternatingly arranged side-by-side in the left-right direction, thus forming a staggered pattern with respect to the up-down direction and the left-right direction.

The area of each fused portion 32a is 0.1 mm² (= 0.5 mm × 0.2 mm). Also, concerning the area ratio of the fused portions 32a, there are 3 fused portions 32a per area of 2.5 mm × 1.5 mm, and thus is (0.1 mm²) × 2/(2.5 mm × 1.5 mm) × 100 = 5.3%.

The front outer-layer sheet 33 is made of spunbond nonwoven fabric as previously described, and is provided with emboss portions (fused portions) that are similar to those of the front inner-layer sheet 31. Accordingly, the area and area ratio of these fused portions (not shown) are the same as those of the fused portions 31a of the front inner-layer sheet 31.

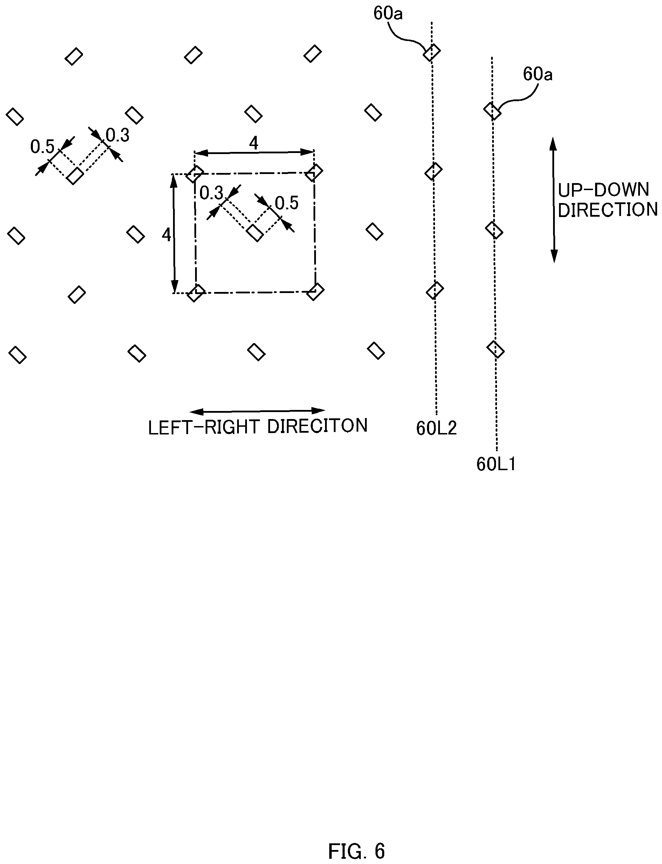

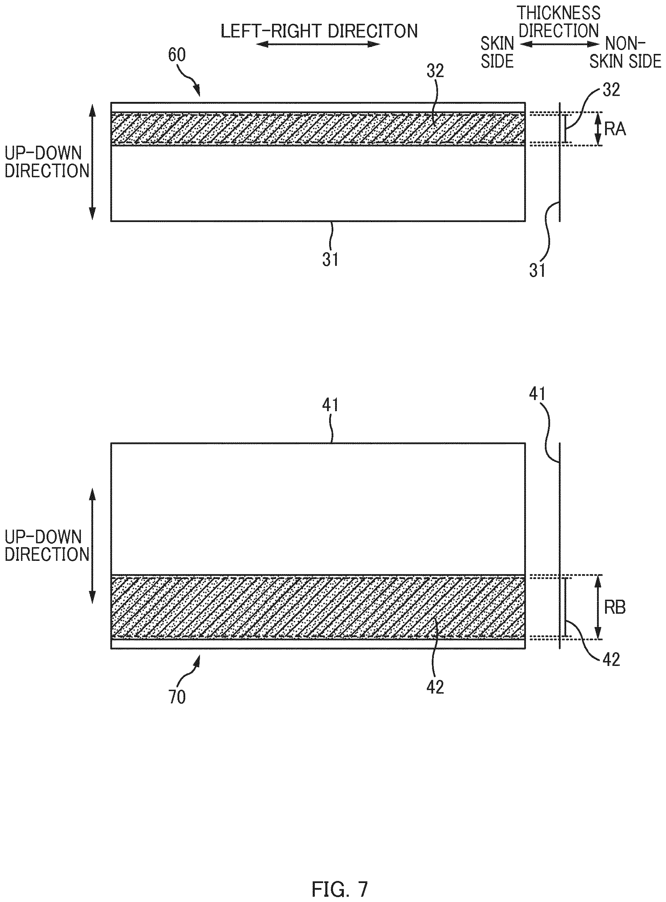

Next, welded portions of the composite sheet 60 will be described. FIG. 6 is a diagram showing an example of a pattern of welded portions 60a formed in the composite sheet 60. FIG. 7 is a diagram for illustrating configurations of the composite sheets 60 and 70. Note that figure on the upper side in FIG. 7 is a front view, and the figure on the lower side in FIG. 7 is a side view. In the composite sheet 60, the sheets that are overlaid in the thickness direction (here, the front inner-layer sheet 31 and the front reinforcing sheet 32) are joined to each other by intermittent welding in the welded portions 60a.

The welded portions 60a are formed in a pattern (with rectangles of long sides of 0.5 mm and short sides of 0.3 mm) that is similar to the fused portions 32a of the front reinforcing sheet 32. The welded portions 60a are arranged side-by-side in the up-down direction to form dot lines 60L1 and dot lines 60L2 in the composite sheet 60. In the dot lines 60L1, the welded portions 60a are arranged side-by-side with gaps therebetween in the up-down direction in a pattern in which the long sides are rotated 45 degrees clockwise from the state of conforming to the left-right direction. In the dot lines 60L2, the welded portions 60a are arranged side-by-side with gaps therebetween in the up-down direction in a pattern in which the long sides are rotated 45 degrees counter-clockwise from the state of conforming to the left-right direction. Note that with respect to the up-down direction, the welded portions 60a in the dot lines 62L0 are located between (at an intermediate position between) welded portions 60a that are adjacent in the up-down direction in the dot lines 60L1. These dot lines 60L1 and dot lines 60L2 are alternatingly arranged side-by-side in the left-right direction, thus forming a staggered pattern with respect to the up-down direction and the left-right direction. In this way, the welded portions 60a are arranged at equal intervals in a uniform staggered pattern, and therefore there are no large non-joined regions, and no concentrated weld regions. Accordingly, uniform strength can be achieved, and rupturing can be suppressed without increasing hardness.

The area of each welded portion 60a is 0.15 mm² (= 0.5 mm × 0.3 mm) . Also, concerning the area ratio of the welded portions 60a, there are 2 welded portions 60a per area of 4 mm × 4 mm, and thus is (0.15 mm²) × 2/(4 mm × 4 mm) × 100 = 1.88%.

In this way, in the present embodiment, the area and the area ratio of the welded portions 60a are smaller than the area and the area ratio of the fused portions 31a of the front inner-layer sheet 31. Accordingly, it is possible to further maintain the soft texture of the sheet, and the fit to the wearer's body can be improved.

Note that the area and the area ratio of the fused portions 31a of the front inner-layer sheet 31 are respectively set larger than the area and the area ratio of the welded portions 60a in the present embodiment, but either the area or the area ratio may be smaller. Also, the area and the area ratio of the fused portions 32a of the front reinforcing sheet 32 may be smaller than at least one of the area and the area ratio of the welded portions 60a. It is desirable that the area and the area ratio of the welded portions 60a are smaller than both the area and the area ratio of the fused portions 31a of the front inner-layer sheet 31, or smaller than both the area and the area ratio of the fused portions 32a of the front reinforcing sheet 32.

The welded portions 60a are formed by performing ultrasonic welding (sonic sealing), heat welding (heat sealing), or the like to perform welding between two sheets that are overlaid in the thickness direction (here, the front inner-layer sheet 31 and the front reinforcing sheet 32). Specifically, while the two sheets are transported in the direction of transport (e.g., the longitudinal direction), welding is performed thereon with use of a protrusion pattern provided on the outer circumferential surface of an emboss roller provided on a rotation shaft that extends in a direction orthogonal to the direction of transport (e.g., in the left-right direction), thus forming the welded portions 60a. Note that in the present embodiment, the welded portions 60a are formed by ultrasonic welding. Ultrasonic welding can maintain the texture better than heat welding. Note that the welded portions 60a are formed such that welding traces remain on the non-skin-side surfaces of the sheets.

As shown in FIG. 7, the front reinforcing sheet 32 has a shorter length in the up-down direction than the front inner-layer sheet 31, and is shaped as a rectangle elongated extending to the two ends in the left-right direction of the front inner-layer sheet 31. The front reinforcing sheet 32 is overlaid on the non-skin-side surface of the front inner-layer sheet 31 and joined thereto by intermittent welding in the welded portions 60a, and thus the composite sheet 60 is formed by the front inner-layer sheet 31 and the front reinforcing sheet 32. Similarly, in the back panel portion 40, the composite sheet 70 is formed by the back inner-layer sheet 41 and the back reinforcing sheet 42.

In the present embodiment, the formation range of the welded portions 60a (formation range in the up-down direction) is set longer than the length of the front reinforcing sheet 32 in the up-down direction. Specifically, in the present embodiment, the welded portions 60a are formed extending approximately 5 mm outward from the two vertical sides of the front reinforcing sheet 32 (the range indicated by RA in FIG. 7) . By forming the welded portions 60a so as to straddle the upper and lower end portions of the front reinforcing sheet 32 in this way, it is possible to prevent twisting and detachment of the front reinforcing sheet 32 during use, and it is possible to reliably reinforce the region in which the front reinforcing sheet 32 is arranged. In the present embodiment, the welded portions 60a are formed so as to straddle the upper and lower end portions of the front reinforcing sheet 32. However, it should be noted that twisting and detachment can be prevented if the welded portions 60a straddle at least either the upper or lower end portion in at least one portion in the left-right direction.

After the composite sheet 60 is formed, the front waist elastic members 34 and the waist elastic members 35, which have an adhesive applied thereto, are arranged on the non-skin-side surface of the composite sheet 60 with gaps therebetween in the up-down direction. And, with the adhesive, joined are the front outer-layer sheet 33 and the composite sheet 60 (the front inner-layer sheet 31 and the front reinforcing sheet 32). Accordingly, the overlaid portion 30s is formed by the front inner-layer sheet 31, the front reinforcing sheet 32, and the front outer-layer sheet 33 in this order from the skin side to the non-skin side in the thickness direction. In this way, in the present embodiment, the sheets are joined to each other by the adhesive applied to the front waist elastic members 34, thus eliminating the need to needlessly apply adhesive. Also, portions not provided with adhesive (portions where the front inner-layer sheet 31 and the composite sheet 60 are not joined) exist between adjacent front waist elastic members 34. Accordingly, the adhesive can be used efficiently. Also, softness can be further improved.

Also, with respect to the up-down direction, at least a portion of the overlaid portion 30s is provided between a stretchy region (corresponding to the first stretchy region) in which the waist elastic members 35 are provided along the edge of the waist opening BH and a stretchy region (corresponding to the second stretchy region) in which the leg elastic members 36 are provided along the edges of the pair of leg openings HL. Accordingly, it is possible to reinforce the portion that is likely to be subjected to force during use.

Also, as previously described, the front outer-layer sheet 33 is folded back downward on the skin side in the thickness direction at the upper end 30eu of the front panel portion 30 (in other words, at the edge of the waist opening BH), thus forming the folded portion 33f. As shown in FIG. 3, the upper end of the overlaid portion 30s (in other words, the upper end of the front reinforcing sheet 32) is located higher than the lower end of the folded portion 33f of the front outer-layer sheet 33. Accordingly, a region having only two sheets (the front inner-layer sheet 31 and the front outer-layer sheet 33) does not exist between the folded portion 33f and the overlaid portion 30s, thus making it possible to more reliably reinforce the region.

Also, in the present embodiment, the upper end of the front reinforcing sheet 32 is located higher than the lowermost waist elastic member 35. In other words, the front reinforcing sheet 32 and the waist elastic members 35 are partially overlapped. Accordingly, it is even more difficult for a finger to enter. Note that the upper end of the front reinforcing sheet 32 may be lower than the lowermost waist elastic member 35. In this case, it is desirable that the gap between the upper end of the front reinforcing sheet 32 and the lowermost waist elastic member 35 is less than or equal to 5 mm. This makes it possible to prevent penetration by a finger.

The configuration on the back panel portion 40 side is similar to that on the front panel portion 30 side. In other words, the back reinforcing sheet 42 is overlaid on the back inner-layer sheet 41, and the welded portions (not shown; similar to the welded portions 60a) are formed extending approximately 5 mm outward from the two vertical sides of the back reinforcing sheet 42 (the range indicated by RB in FIG. 7). Accordingly, the back inner-layer sheet 41 and the back reinforcing sheet 42 are intermittently welded in the welded portions, thus forming the composite sheet 70. The back waist elastic members 44, which have an adhesive applied thereto, are then arranged on the non-skin-side surface of the composite sheet 70 with gaps therebetween in the up-down direction, and the back outer-layer sheet 43 and the composite sheet 70 (the back inner-layer sheet 41 and the back reinforcing sheet 42) are joined via the adhesive. Accordingly, the overlaid portion 40s is formed by the back inner-layer sheet 41, the back reinforcing sheet 42, and the back outer-layer sheet 43 in this order from the skin side to the non-skin side in the thickness direction.

Note that in the present embodiment, as shown in FIGS. 2 and 3, the length of the overlaid portion 40s in the up-down direction (in other words, the length of the back reinforcing sheet 42 in the up-down direction) is set larger than the length of the overlaid portion 30s in the up-down direction (in other words, the length of the front reinforcing sheet 32 in the up-down direction). Specifically, the lower end of the overlaid portion 30s is located higher than the center of the front waist portion 1A in the up-down direction, whereas the lower end of the overlaid portion 40s is located lower than the center of the back waist portion 1C in the up-down direction. This is because the back side has a large region that is easily grabbed with fingers when the diaper is pulled up (pull-up is difficult due to the protruding buttocks portion). Accordingly, this region can be effectively reinforced.

### Other embodiments

Although the embodiment of the present disclosure have been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof. For example, modification which will be described below is possible.

An example of using elastic strings as the elastic members such as the front waist elastic members 34 is described in the above embodiment, but these elastic members are not limited to being string-like elastic members such as so-called elastic strings. For example, flat (band-shaped) elastic members having a predetermined width may be used.

In the embodiment described above, the overlaid portion 30s and the overlaid portion 40s are respectively provided in the front panel portion 30 and the back panel portion 40 to reinforce both the front side and the back side, but a configuration is possible in which only one side is reinforced. For example, a configuration is possible in which only the back side (the back panel portion 40 side) is reinforced. Also, although a portion close to the waist opening BH is reinforced in the embodiment described above, portions close to the pair of leg openings LH may be reinforced. In other words, the leg elastic members 36 and 46 may be used as elastic members in the overlaid portions 30s and 40s. In this case as well, the sheets may be joined to each other by applying an adhesive to regions including the leg elastic members 36 and 46. Accordingly, it is possible to maintain the strength and texture of the crotch portion 1B.

Although the reinforcing sheets (e.g., the front reinforcing sheet 32) are arranged between the inner-layer sheets (e.g., the front inner-layer sheet 31) and the outer-layer sheets (e.g., the front outer-layer sheet 33) in the embodiment described above, a configuration is possible in which a reinforcing sheet is arranged as the skin side (inward) sheet. In this case, the reinforcing sheet corresponds to the first sheet member, and the inner-layer sheet corresponds to the second sheet member. Also, in this case, it is further desirable that the welded portions that join the reinforcing sheet and the inner-layer sheet are formed so as to straddle the upper and lower ends of the reinforcing sheet. Accordingly, it is possible to prevent the reinforcing sheet from peeling, and it is possible to reliably maintain strength up to the ends in the up-down direction.

In the region of the front panel portion 30 that is overlapped with the absorbent main body 10, the area and the area ratio of the welded portions 60a may be smaller than in the other regions (a similar configuration is applied in the back panel portion 40 as well) . The region overlapped with the absorbent main body 10 is not likely to become torn during use, and therefore the area and the area ratio of the welded portions can be reduced, thus making it possible to further improve the softness.

In each of the composite sheets (e.g., the composite sheet 60), either one of the two constituent sheets (e.g., the front inner-layer sheet 31 and the front reinforcing sheet 32) may be constituted by stretchable nonwoven fabric. In the case where the inward sheet (inward inner-layer sheet 31) is made of stretchable nonwoven fabric, the front reinforcing sheet 32 can be used to supplement the insufficiency in strength arising from one stretchable nonwoven fabric, and the effects of the stretchable nonwoven fabric can be achieved. It is also possible to reduce the material cost.

Also, in the case where the intermediate sheet (front reinforcing sheet 32) is made of stretchable nonwoven fabric, it is possible to achieve a soft texture without impairing the texture and stretchability of the elastic material.

The shape of the emboss portions (fused portions) formed on the surfaces of the sheets, and the shape of the pattern of welded portions for joining sheets to each other are not limited to the shapes described above. For example, square, circular, or polygonal shapes may be employed. Also, the pattern arrangement (alignment) in the up-down direction and the left-right direction is not required to be a staggered pattern. For example, a lattice pattern may be employed.

### [Reference Signs List]

- 1: pull-on disposable diaper (pants-shaped absorbent article),
- 1A: front waist portion, 1B crotch portion, 1C back waist portion,
- 10: absorbent main body,
- 11: top sheet, 12 second sheet,
- 13: absorbent core, 13a upper layer core, 13b lower layer core,
- 14: back sheet, 15 crotch-elastic-member covering sheet,
- 16: crotch elastic portion, 161 crotch elastic member,
- 20: crotch exterior portion (exterior member),
- 30: front panel portion (exterior member), 30s overlaid portion,
- 30es: front side joining portion, 30eu upper end,
- 31: front inner-layer sheet (first sheet member), 31a fused portion,
- 32: front reinforcing sheet (second sheet member), 32a fused portion,
- 33: front outer-layer sheet (third sheet member), 33f folded portion,
- 34: front waist elastic member (elastic member),
- 35: waist elastic member,
- 36: leg elastic member,
- 40: back panel portion (exterior member), 40s overlaid portion,
- 40es: back side joining portion, 40eu upper end,
- 41: back inner-layer sheet (first sheet member),
- 42: back reinforcing sheet (second sheet member),
- 43: back outer-layer sheet (third sheet member), 43f folded portion,
- 44: back waist elastic member (elastic member),
- 45: waist elastic member,
- 46: leg elastic member,
- 60: composite sheet, 60a welded portion,
- 70: composite sheet,
- BH: waist opening, LH leg opening,
- CL10: predetermined position

## Claims

1. A pants-shaped absorbent article (1)
having an up-down direction, a left-right direction, and a front-back direction that intersect each other, and
having a waist opening (BH) and a pair of leg openings (LH),
the pants-shaped absorbent article (1) comprising:
an absorbent main body (10) having an absorbent core (13); and
an exterior member (30) joined to the absorbent main body (10),
the exterior member (30) having an overlaid portion (30s) in which a first sheet member (31), a second sheet member (32), and a third sheet member (33) are overlaid in order from a skin side to a non-skin side in a thickness direction,
the first sheet member (31) and the second sheet member (32) being joined by being intermittently welded in a plurality of welded portions (60a),
an elastic member (34) being arranged between the second sheet member (32) and the third sheet member (33),
the second sheet member (32) and the third sheet member (33) being joined using an adhesive; wherein
a length of the second sheet member (32) in the up-down direction is shorter than a length of the first sheet member (31) in the up-down direction, and
the plurality of welded portions (60a) are formed so as to straddle at least one end portion in the up-down direction of the second sheet member (32) in a portion in the left-right direction.

2. A pants-shaped absorbent article (1) according to claim 1, wherein
the plurality of welded portions (60a) are arranged in a staggered manner in the up-down direction and the left-right direction.

3. A pants-shaped absorbent article (1) according to claim 1 or claim 2, wherein
the first sheet member (31) and the second sheet member (32) each have a plurality of fused portions at a surface, and
a ratio of an area of the welded portions (60a) provided per unit area of the overlaid portion (30s)
is smaller than at least either one of
a ratio of an area of the fused portions (31a) provided per unit area of the first sheet member (31) and
a ratio of an area of the fused portions (32a) provided per unit area of the second sheet member (32).

4. A pants-shaped absorbent article (1) according to any one of claims 1 to 3, wherein
the first sheet member (31) and the second sheet member (32) each have a plurality of fused portions at a surface, and
an area of each of the welded portions (60a)
is smaller than at least either one of
an area of each of the fused portions (31a) in the first sheet member (31) and
an area of each of the fused portions (32a) in the second sheet member (32).

5. A pants-shaped absorbent article (1) according to any one of claims 1 to 4, wherein
a ratio of an area of the welded portions (60a) provided per unit area of a region in which the exterior member (30) and the absorbent main body (10) are overlapped
is smaller than
a ratio of an area of the welded portions provided per unit area of another region.

6. A pants-shaped absorbent article (1) according to any one of claims 1 to 5, wherein
a plurality of the elastic members (34) are arranged with gaps in the up-down direction,
the adhesive is applied to each of the elastic members (34), and
a portion in which the second sheet member (32) and the third sheet member (33) are not joined by the adhesive is provided between adjacent elastic members (34).

7. A pants-shaped absorbent article (1) according to any one of claims 1 to 6, wherein
the exterior member (30) includes
a first stretchy region that extends along an edge of the waist opening (BH) and
a pair of second stretchy regions that respectively extend along edges of the pair of leg openings (LH), and
with respect to the up-down direction, at least a portion of the overlaid portion (30s) is provided between the first stretchy region and the pair of second stretchy regions.

8. A pants-shaped absorbent article (1) according to claim 7, wherein
a plurality of waist elastic members (35) that are arranged with gaps in the up-down direction is provided in the first stretchy region, and
a gap between adjacent elastic members (34) in the overlaid portion (30s) is larger than a gap between adjacent waist elastic members (35) in the first stretchy region.

9. A pants-shaped absorbent article (1) according to any one of claims 1 to 8, wherein
the exterior member (30) includes
a front waist portion (1A) that is to be arranged on a stomach side of a wearer, and
a back waist portion (1C) that is to be arranged on a back side of the wearer, and
the overlaid portion (30s) is provided in at least either one of the front waist portion (1A) and the back waist portion (1C).

10. A pants-shaped absorbent article (1) according to claim 9, wherein
the overlaid portion is provided in both the front waist portion (1A) and the back waist portion (1C), and
an up-down-direction length of the overlaid portion (40s) of the back waist portion (1C) is larger than an up-down-direction length of the overlaid portion (30s) of the front waist portion (1A).

11. A pants-shaped absorbent article (1) according to any one of claims 1 to 10, wherein
the third sheet member (33) is folded back downward on the skin side in the thickness direction at an edge (30eu) of the waist opening (BH), and
an upper end of the second sheet member (32) in the up-down direction is located higher than a lower end of a folded portion (33f) of the third sheet member (33).

12. A method for manufacturing a pants-shaped absorbent article (1) having a waist opening (BH) and a pair of leg openings (LH),
the pants-shaped absorbent article (1) including:
an absorbent main body (10) having an absorbent core (13); and
an exterior member (30) joined to the absorbent main body (10),
the method comprising:
intermittently welding a first sheet member (31) and a second sheet member (32) with a plurality of welded portions (60a);
arranging an elastic member (34) between a surface of the second sheet member (32) and a third sheet member (33) and joining the second sheet member (32) and the third sheet member (33) with an adhesive,
the surface of the second sheet member (32) being located on an opposite side of the first sheet member (31); and
forming an overlaid portion (30s) in which the first sheet member (31), the second sheet member (32), and the third sheet member (33) are overlaid in order from a skin side to a non-skin side in a thickness direction of the exterior member (30); wherein
the absorbent article (1) is according to any one of claims 1 to 11.

## Patentansprüche

1. Höschenförmiger absorbierender Artikel (1),
der eine Oben-unten-Richtung, eine Links-rechts-Richtung und eine Vorn-hinten-Richtung aufweist, die einander schneiden, und
der eine Taillenöffnung (BH) und ein Paar von Beinöffnungen (LH) aufweist,
wobei der höschenförmige absorbierende Artikel (1) Folgendes umfasst:
einen absorbierenden Hauptkörper (10), der einen Saugkern (13) aufweist; und
ein äußeres Element (30), das mit dem absorbierenden Hauptkörper (10) verbunden ist,
wobei das äußere Element (30) einen überlagerten Teil (30s) aufweist, in dem ein erstes Lagenelement (31), ein zweites Lagenelement (32) und ein drittes Lagenelement (33) in der Reihenfolge von einer Hautseite zu einer Nicht-Hautseite in einer Dickenrichtung überlagert sind,
das erste Lagenelement (31) und das zweite Lagenelement (32) durch intermittierendes Verschweißen in einer Vielzahl von verschweißten Teilen (60a) verbunden sind,
ein elastisches Element (34) zwischen dem zweiten Lagenelement (32) und dem dritten Lagenelement (33) angeordnet ist,
das zweite Lagenelement (32) und das dritte Lagenelement (33) unter Verwendung eines Haftmittels verbunden sind; wobei
eine Länge des zweiten Lagenelements (32) in der Oben-unten-Richtung kürzer ist als eine Länge des ersten Lagenelements (31) in der Oben-und-Richtung und
die Vielzahl von verschweißten Teilen (60a) derart ausgebildet ist, dass sie mindestens einen Endteil in der Oben-unten-Richtung des zweiten Lagenelements (32) in einem Teil der Links-rechts-Richtung überspannt.

2. Höschenförmiger absorbierender Artikel (1) nach Anspruch 1, wobei
die Vielzahl von verschweißten Teilen (60a) in einer versetzten Weise in der Oben-unten-Richtung und der Links-rechts-Richtung angeordnet ist.

3. Höschenförmiger absorbierender Artikel (1) nach Anspruch 1 oder Anspruch 2, wobei
das erste Lagenelement (31) und das zweite Lagenelement (32) jeweils eine Vielzahl von fusionierten Teilen an einer Oberfläche aufweisen und
ein Verhältnis einer Fläche der verschweißten Teile (60a), die pro Einheitsfläche des überlagerten Teils (30s) bereitgestellt sind,
kleiner ist als mindestens eines von
einem Verhältnis einer Fläche der fusionierten Teile (31a), die pro Einheitsfläche des ersten Lagenelements (31) bereitgestellt sind, und
einem Verhältnis einer Fläche der fusionierten Teile (32a), die pro Einheitsfläche des zweiten Lagenelements (32) bereitgestellt sind.

4. Höschenförmiger absorbierender Artikel (1) nach einem der Ansprüche 1 bis 3, wobei
das erste Lagenelement (31) und das zweite Lagenelement (32) jeweils eine Vielzahl von fusionierten Teilen an einer Oberfläche aufweisen und
eine Fläche von jedem der verschweißten Teile (60a)
kleiner ist als mindestens eine von
einer Fläche von jedem der fusionierten Teile (31a) in dem ersten Lagenelement (31) und
einer Fläche von jedem der fusionierten Teile (32a) in dem zweiten Lagenelement (32).

5. Höschenförmiger absorbierender Artikel (1) nach einem der Ansprüche 1 bis 4, wobei
ein Verhältnis einer Fläche der verschweißten Teile (60a), die pro Einheitsfläche eines Bereichs bereitgestellt sind, in dem das äußere Element (30) und der absorbierende Hauptkörper (10) überlappt sind,
kleiner ist als
ein Verhältnis einer Fläche der verschweißten Teile, die pro Einheitsfläche des anderen Bereichs bereitgestellt sind.

6. Höschenförmiger absorbierender Artikel (1) nach einem der Ansprüche 1 bis 5, wobei
eine Vielzahl der elastischen Elemente (34) mit Lücken in der Oben-unten-Richtung angeordnet ist,
das Haftmittel auf jedes der elastischen Elemente (34) aufgetragen ist und
ein Teil, in dem das zweite Lagenelement (32) und das dritte Lagenelement (33) nicht durch das Haftmittel verbunden sind, zwischen benachbarten elastischen Elementen (34) bereitgestellt ist.

7. Höschenförmiger absorbierender Artikel (1) nach einem der Ansprüche 1 bis 6, wobei
das äußere Element (30) Folgendes einschließt:
einen ersten dehnbaren Bereich, der sich entlang eines Rands der Taillenöffnung (BH) erstreckt, und
ein Paar von zweiten dehnbaren Bereichen, die sich jeweils entlang der Ränder des Paars von Beinöffnungen (LH) erstrecken, und,
in Bezug auf die Oben-und-Richtung, mindestens ein Teil des überlagerten Teils (30s) zwischen dem ersten dehnbaren Bereich und dem Paar von zweiten dehnbaren Bereichen bereitgestellt ist.

8. Höschenförmiger absorbierender Artikel (1) nach Anspruch 7, wobei
eine Vielzahl von elastischen Taillenelementen (35), die mit Lücken in der Oben-unten-Richtung angeordnet sind, in dem ersten dehnbaren Bereich bereitgestellt ist und
eine Lücke zwischen benachbarten elastischen Elementen (34) in dem überlagerten Teil (30s) größer ist als eine Lücke zwischen benachbarten elastischen Taillenelementen (35) in dem ersten dehnbaren Bereich.

9. Höschenförmiger absorbierender Artikel (1) nach einem der Ansprüche 1 bis 8, wobei
das äußere Element (30) Folgendes einschließt:
einen vorderen Taillenteil (1A), der auf einer Bauchseite eines Trägers anzuordnen ist, und
einen hinteren Taillenteil (1C), der auf einer Rückenseite des Trägers anzuordnen ist, und
der überlagerte Teil (30s) in zumindest einem von dem vorderen Taillenteil (1A) und dem hinteren Taillenteil (1C) bereitgestellt ist.

10. Höschenförmiger absorbierender Artikel (1) nach Anspruch 9, wobei
der überlagerte Teil in sowohl dem vorderen Taillenteil (1A) als auch dem hinteren Taillenteil (1C) bereitgestellt ist und
eine Länge in der Oben-unten-Richtung des überlagerten Teils (40s) des hinteren Taillenteils (1C) größer ist als eine Länge in der Oben-unten-Richtung des überlagerten Teils (30s) des vorderen Taillenteils (1A).

11. Höschenförmiger absorbierender Artikel (1) nach einem der Ansprüche 1 bis 10, wobei
das dritte Lagenelement (33) nach unten auf der Hautseite in der Dickenrichtung an einem Rand (30eu) der Taillenöffnung (BH) zurückgefaltet ist und
ein oberes Ende des zweiten Lagenelements (32) in der Oben-unten-Richtung höher positioniert ist als ein unteres Ende eines gefalteten Teils (33f) des dritten Lagenelements (33).

12. Verfahren für die Herstellung eines höschenförmigen absorbierenden Artikels (1), der eine Taillenöffnung (BH) und ein Paar von Beinöffnungen (LH) aufweist,
wobei der höschenförmige absorbierende Artikel (1) Folgendes einschließt:
einen absorbierenden Hauptkörper (10), der einen Saugkern (13) aufweist; und
ein äußeres Element (30), das mit dem absorbierenden Hauptkörper (10) verbunden ist,
wobei das Verfahren Folgendes umfasst:
intermittierendes Verschweißen eines ersten Lagenelements (31) und eines zweiten Lagenelements (32) mit einer Vielzahl von verschweißten Teilen (60a);
Anordnen eines elastischen Elements (34) zwischen einer Oberfläche des zweiten Lagenelements (32) und einem dritten Lagenelement (33) und Verbinden des zweiten Lagenelements (32) und des dritten Lagenelements (33) mit einem Haftmittel,
wobei die Oberfläche des zweiten Lagenelements (32) auf einer gegenüberliegenden Seite des ersten Lagenelements (31) positioniert wird; und
Ausbilden eines überlagerten Teils (30s), in dem das erste Lagenelement (31), das zweite Lagenelement (32) und das dritte Lagenelement (33) in der Reihenfolge von einer Hautseite zu einer Nicht-Hautseite in einer Dickenrichtung des äußeren Elements (30) überlagert sind; wobei
der absorbierende Artikel (1) gemäß einem der Ansprüche 1 bis 11 vorliegt.

## Revendications

1. Article absorbant en forme de culotte (1)
ayant une direction allant de haut en bas, une direction allant de gauche à droite, et une direction allant d'avant en arrière qui se croisent les unes les autres, et
ayant une ouverture au niveau de la taille (BH) et une paire d'ouvertures au niveau des jambes (LH),
l'article absorbant en forme de culotte (1) comportant :
un corps principal absorbant (10) ayant une partie centrale absorbante (13) ; et
un élément extérieur (30) assemblé au niveau du corps principal absorbant (10),
l'élément extérieur (30) ayant une partie superposée (30s) dans laquelle un premier élément formant feuille (31), un deuxième élément formant feuille (32), et un troisième élément formant feuille (33) sont superposés dans l'ordre depuis un côté peau jusque sur un côté non-peau dans une direction allant dans le sens de l'épaisseur,
le premier élément formant feuille (31) et le deuxième élément formant feuille (32) étant assemblés en étant soudés de manière intermittente en une pluralité de parties soudées (60a),
un élément élastique (34) étant agencé entre le deuxième élément formant feuille (32) et le troisième élément formant feuille (33),
le deuxième élément formant feuille (32) et le troisième élément formant feuille (33) étant assemblés en utilisant un adhésif ; dans lequel
une longueur du deuxième élément formant feuille (32) dans la direction allant de haut en bas est plus courte qu'une longueur du premier élément formant feuille (31) dans la direction allant de haut en bas, et
les parties soudées de la pluralité de parties soudées (60a) sont formées de manière à enjamber au moins une partie d'extrémité dans la direction allant de haut en bas du deuxième élément formant feuille (32) dans une partie dans la direction allant de gauche à droite.

2. Article absorbant en forme de culotte (1) selon la revendication 1, dans lequel
les parties soudées de la pluralité de parties soudées (60a) sont agencées d'une manière décalée dans la direction allant de haut en bas et dans la direction allant de gauche à droite.

3. Article absorbant en forme de culotte (1) selon la revendication 1 ou la revendication 2, dans lequel
le premier élément formant feuille (31) et le deuxième élément formant feuille (32) ont chacun une pluralité de parties thermofixées au niveau d'une surface, et
un rapport d'une surface des parties soudées (60a) fourni par surface unitaire de la partie superposée (30s)
est inférieur à au moins l'un quelconque parmi
un rapport d'une surface des parties thermofixées (31a) fourni par surface unitaire du premier élément formant feuille (31) et
un rapport d'une surface des parties thermofixées (32a) fourni par surface unitaire du deuxième élément formant feuille (32).

4. Article absorbant en forme de culotte (1) selon l'une quelconque des revendications 1 à 3, dans lequel
le premier élément formant feuille (31) et le deuxième élément formant feuille (32) ont chacun une pluralité de parties thermofixées au niveau d'une surface, et
une surface de chacune des parties soudées (60a)
est inférieure à au moins l'une quelconque parmi
une surface de chacune des parties thermofixées (31a) dans le premier élément formant feuille (31) et
une surface de chacune des parties thermofixées (32a) dans le deuxième élément formant feuille (32).

5. Article absorbant en forme de culotte (1) selon l'une quelconque des revendications 1 à 4, dans lequel
un rapport d'une surface des parties soudées (60a) fourni par surface unitaire d'une région dans laquelle l'élément extérieur (30) et le corps principal absorbant (10) se chevauchent
est inférieur à
un rapport d'une surface des parties soudées fourni par surface unitaire d'une autre région.

6. Article absorbant en forme de culotte (1) selon l'une quelconque des revendications 1 à 5, dans lequel
les éléments élastiques d'une pluralité d'éléments élastiques (34) sont agencés avec des espaces dans la direction allant de haut en bas,
l'adhésif est appliqué sur chacun des éléments élastiques (34), et
une partie dans laquelle le deuxième élément formant feuille (32) et le troisième élément formant feuille (33) ne sont pas assemblés par l'adhésif est mise en œuvre entre des éléments élastiques adjacents (34).

7. Article absorbant en forme de culotte (1) selon l'une quelconque des revendications 1 à 6, dans lequel
l'élément extérieur (30) comprend
une première région extensible qui s'étend le long d'un bord de l'ouverture au niveau de la taille (BH) et
une paire de deuxièmes régions extensibles qui s'étendent respectivement le long des bords de la paire d'ouvertures au niveau des jambes (LH), et
par rapport à la direction allant de haut en bas, au moins une partie de la partie superposée (30s) est mise en œuvre entre la première région extensible et la paire de deuxièmes régions extensibles.

8. Article absorbant en forme de culotte (1) selon la revendication 7, dans lequel
une pluralité d'éléments élastiques au niveau de la taille (35) qui sont agencés avec des espaces dans la direction allant de haut en bas est mise en œuvre dans la première région extensible, et
un espace entre des éléments élastiques adjacents (34) dans la partie superposée (30s) est supérieur à un espace entre des éléments élastiques adjacents au niveau de la taille (35) dans la première région extensible.

9. Article absorbant en forme de culotte (1) selon l'une quelconque des revendications 1 à 8, dans lequel
l'élément extérieur (30) comprend
une partie avant au niveau de la taille (1A) qui est destinée à être agencée sur un côté estomac d'un utilisateur, et
une partie arrière au niveau de la taille (1C) qui est destinée à être agencée sur un côté dos de l'utilisateur, et
la partie superposée (30s) est mise en œuvre dans au moins l'une quelconque parmi la partie avant au niveau de la taille (1A) et la partie arrière au niveau de la taille (1C).

10. Article absorbant en forme de culotte (1) selon la revendication 9, dans lequel
la partie superposée est mise en œuvre à la fois dans la partie avant au niveau de la taille (1A) et la partie arrière au niveau de la taille (1C), et
une longueur dans la direction allant de haut en bas de la partie superposée (40s) de la partie arrière au niveau de la taille (1C) est plus longue qu'une longueur dans la direction allant de haut en bas de la partie superposée (30s) de la partie avant au niveau de la taille (1A).

11. Article absorbant en forme de culotte (1) selon l'une quelconque des revendications 1 à 10, dans lequel
le troisième élément formant feuille (33) est replié vers le bas sur le côté peau dans la direction allant dans le sens de l'épaisseur au niveau d'un bord (30eu) de l'ouverture au niveau de la taille (BH), et
une extrémité supérieure du deuxième élément formant feuille (32) dans la direction allant de haut en bas se trouve plus haut par rapport à une extrémité inférieure d'une partie pliée (33f) du troisième élément formant feuille (33).

12. Procédé de fabrication d'un article absorbant en forme de culotte (1) ayant une ouverture au niveau de la taille (BH) et une paire d'ouvertures au niveau des jambes (LH),
l'article absorbant en forme de culotte (1) comprenant :
un corps principal absorbant (10) ayant une partie centrale absorbante (13) ; et
un élément extérieur (30) assemblé au niveau du corps principal absorbant (10),
le procédé comportant les étapes consistant à :
souder de manière intermittente un premier élément formant feuille (31) et un deuxième élément formant feuille (32) avec une pluralité de parties soudées (60a) ;
agencer un élément élastique (34) entre une surface du deuxième élément formant feuille (32) et un troisième élément formant feuille (33) et assembler le deuxième élément formant feuille (32) et le troisième élément formant feuille (33) au moyen d'un adhésif,
la surface du deuxième élément formant feuille (32) étant située sur un côté opposé du premier élément formant feuille (31) ; et
former une partie superposée (30s) dans laquelle le premier élément formant feuille (31), le deuxième élément formant feuille (32), et le troisième élément formant feuille (33) sont superposés dans l'ordre depuis un côté peau jusque sur un côté non-peau dans une direction allant dans le sens de l'épaisseur de l'élément extérieur (30) ; dans lequel
l'article absorbant (1) est selon l'une quelconque des revendications 1 à 11.
